# EUROPEAN PATENT APPLICATION

(11) **EP 3 954 435 A1**
(43) Date of publication of application: **16.02.2022**
(21) Application number: 20787381.1
(22) Date of filing: 08.04.2020
(51) Int. Cl.: A61P 1/16, A61P 11/00, A61P 29/00, A61K 9/06, A61K 9/08, A61K 9/10, A61K 9/12, A61K 9/14, A61K 9/16, A61K 9/20, A61K 9/48, A61K 35/12, A61K 35/42

(54) **THERAPEUTIC AGENT FOR FIBROSIS, INFLAMMATION, AND/OR AGING DISEASE**

(30) Priority: 09.04.2019 JP 2019074263
(71) Applicant: International Space Medical Co., Ltd., Tokyo, 102-0082 (JP)
(72) Inventor: OCHIYA Takahiro, Tokyo 104-0053 (JP); KUWANO Kazuyoshi, Tokyo 104-0053 (JP); FUJITA Yu, Chiba-shi, Chiba 266-0032 (JP); KADOTA Tsukasa, Tokyo 105-0004 (JP)
(74) Representative: Griffin, Philippa Jane
(86) International application number: PCT/JP2020/015861
(87) International publication number: WO 2020/209304

(57) **Abstract**

In an embodiment, an object of the present invention is to provide a therapeutic and/or preventive agent for a fibrosis such as pulmonary fibrosis, an inflammatory disease and/or an aging disease. In an embodiment, the present invention relates to a therapeutic and/or preventive agent for at least one disease of a fibrosis, an inflammatory disease and an aging disease, comprising extracellular vesicle derived from cell of a tissue or a surrounding tissue thereof where disease can occur, in which the cell is differentiated cell, and relates to a pharmaceutical composition containing the therapeutic and/or preventive agent.

## Description

### Technical Field

In an embodiment, the present invention relates to a therapeutic and/or preventive agent for at least one disease of a fibrosis, an inflammatory disease and an aging disease and a pharmaceutical composition containing the therapeutic and/or preventive agent.

### Background Art

Fibrosis of a tissue refers to excessive formation of a fibrous connective tissue and occurs during repair or healing process of an organ or a tissue. Fibrosis of a tissue can occur in various organs. As the fibrosis, for example, pulmonary fibrosis and cirrhosis are known. There are hundreds of thousands of patients having fibroses in this country, but effective treatment methods have not yet been found.

Pulmonary fibrosis is a typical fibrosis, which can be also referred to as an inflammatory disease or an aging disease. Among pulmonary fibroses, idiopathic pulmonary fibrosis (IPF) of an unknown cause is a disease having an extremely poor prognosis; that is, an average life-span after diagnosis is 3 to 5 years. The prevalence rate of IPF in Japan is about 20 per 100,000 and the frequency of the disease in the elderly over 70 years old is conceivably 10 times as high as the rate. For treating pulmonary fibrosis, antifibrotic agents such as pirfenidone and nintedanib, steroids and immunosuppressant agents are used (Non Patent Literatures 1 and 2) but the effects of these agents are limited.

### Citation List

### Non Patent Literatures

Non Patent Literature 1: Azuma A, et al., Am. J. Respir. Crit. Care Med., 2005, 171, 1040-1047.
Non Patent Literature 2: Richeldi L et al., N. Engl. J. Med., 2011, 22, 365 (12), 1079-1087.

### Summary of Invention

### Technical Problem

In an embodiment, an object of the present invention is to provide a therapeutic and/or preventive agent for a fibrosis such as pulmonary fibrosis, an inflammatory disease and/or an aging disease.

### Solution to Problem

The present inventors found that extracellular vesicle derived from cell of a tissue or a surrounding tissue thereof where a disease as mentioned above can occur has an excellent therapeutic and/or preventive effect on the disease, and accomplished the present invention.

The present invention includes the following embodiments.
[1] A therapeutic and/or preventive agent for at least one disease of a fibrosis, an inflammatory disease and an aging disease, comprising extracellular vesicle derived from cell of a tissue or a surrounding tissue thereof where the disease can occur, wherein the cell is differentiated cell.
[2] The therapeutic and/or preventive agent according to [1], wherein the tissue from which the cell donating the extracellular vesicle is derived is different from a tissue having cell receiving the vesicle.
[3] The therapeutic and/or preventive agent according to [1], wherein the cell is epithelial cell, endothelial cell, mesothelial cell, muscle cell or nerve cell.
[4] The therapeutic and/or preventive agent according to [3], wherein the epithelial cell is bronchial epithelial cell, small airway epithelial cell or alveolar epithelial cell.
[5] The therapeutic and/or preventive agent according to [1], wherein the tissue or surrounding tissue is selected from the group consisting of lung or airway; eye; kidney or glomerulus; liver, gallbladder or bile duct; intestinal tract; pancreas; heart; blood vessel; thyroid; brain or nerve; intraperitoneal; uterus; skin; muscle; bone; and joint.
[6] The therapeutic and/or preventive agent according to [5], wherein the tissue or surrounding tissue thereof is lung or airway.
[7] The therapeutic and/or preventive agent according to [4] or [6], wherein the disease is selected from the group consisting of scleroderma, pulmonary fibrosis, COPD (chronic obstructive pulmonary disease), bronchial asthma, ARDS (acute respiratory distress syndrome), pulmonary hypertension, radioactive pneumonitis, lung sarcoidosis, alveolar bleeding accompanied by collagenosis, interstitial pneumonia, bronchiectasis, cystic fibrosis or bronchopulmonary dysplasia.
[8] The therapeutic and/or preventive agent according to any of [1] to [7], wherein the cell is derived from a healthy subject.
[9] The therapeutic and/or preventive agent according to any of [1] to [8], wherein the extracellular vesicle is exosome.
[10] A pharmaceutical composition comprising the therapeutic and/or preventive agent according to any of [1] to [9], as an active ingredient.
[11] The pharmaceutical composition according to [10], wherein the composition is formulated for inhalation, aerosol, injection, intravenous injection, oral, transdermal, transnasal, local, transvaginal, transmucosal or transrectal administration.

The present description includes part or all of the contents as disclosed in Japanese Patent Application No. 2019-074263, which is a priority document of the present application.

### Advantageous Effects of Invention

The present invention provides a therapeutic and/or preventive agent for a fibrosis, an inflammatory disease and/or an aging disease.

### Brief Description of Drawings

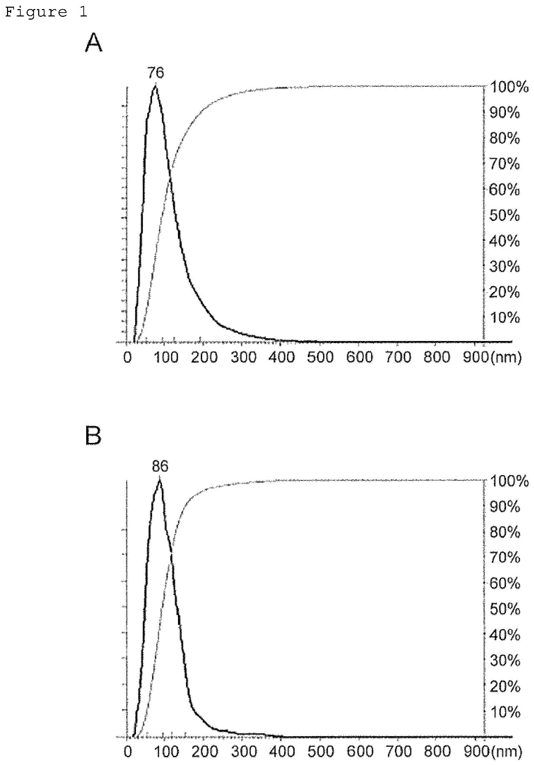

[Figure 1] Figure 1 shows the NTA analysis results (A: HBEC, B: BEAS-2B) of exosome ultracentrifugally collected from the culture supernatant of human primary bronchial epithelial cell HBEC and human bronchial epithelial cell line BEAS-2B. The thick lines each represent the percentage of exosome at individual size. The numeral values (A: 76 nm, B: 86 nm) represent size of exosome highly frequently present. The thin lines each represent the accumulated total.

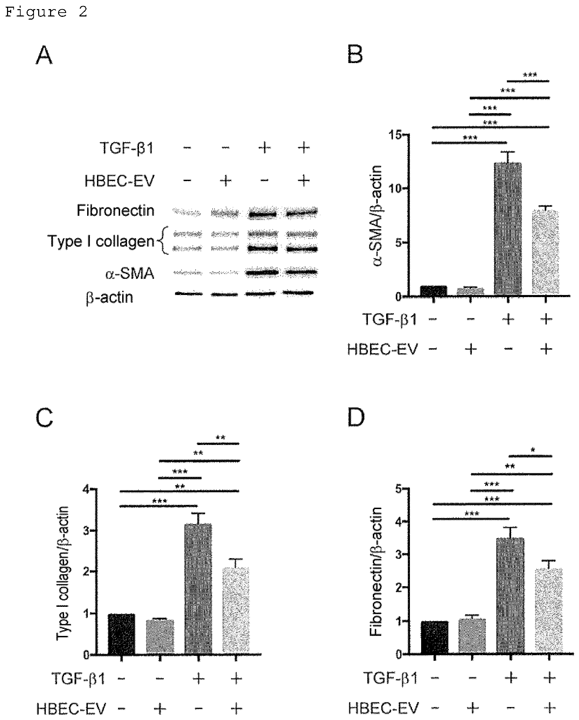

[Figure 2] Figure 2A shows the expression results of various myofibroblast markers confirmed by western blotting after primary lung fibroblast was stimulated with TGF-β1 and/or exosome. Figure 2B to D shows the relative values obtained by dividing the expression levels of individual protein by that of β-actin. Reference symbol * shows a significant difference (*: p < 0.05, **: p < 0.01, ***: p < 0.001). EV stands for extracellular vesicle.

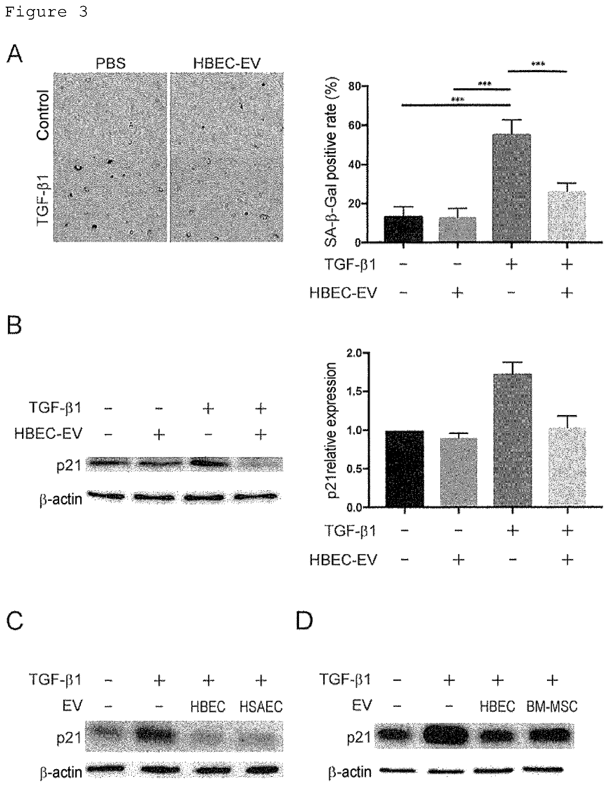

[Figure 3] Figure 3 shows (A) SA-β-Gal positive rate and (B-D) relative expression levels of p21 after primary lung fibroblast was stimulated with TGF-β1 and/or exosome (human bronchial epithelial cell HBEC-derived exosome, human small airway epithelial cell HSAEC-derived exosome or bone marrow-derived mesenchymal stem cell BM-MSC-derived exosome). Reference symbol * shows a significant difference (***: p < 0.001).

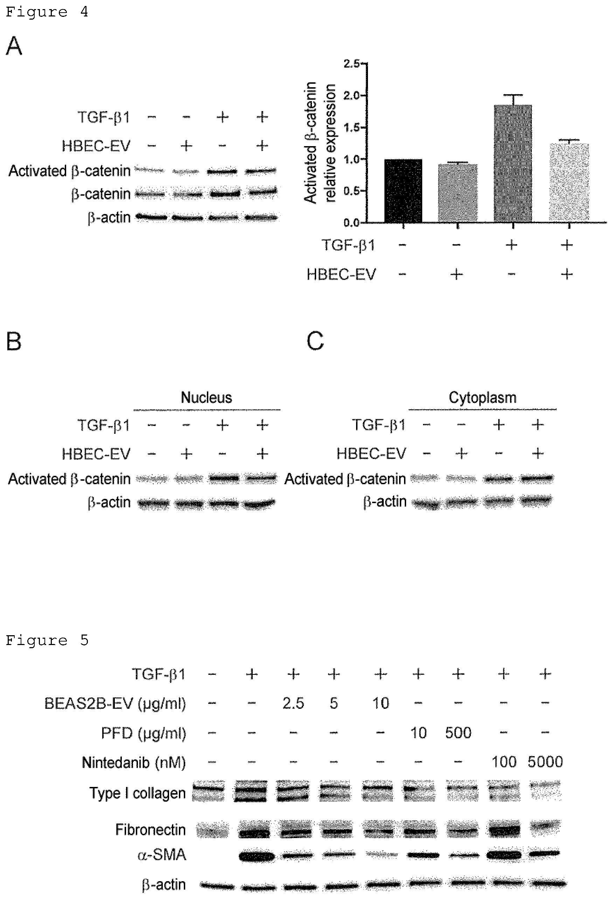

[Figure 4] Figure 4 shows the expression of activated β-catenin confirmed by western blotting after bronchial epithelial cell was stimulated with TGF-β1 and/or exosome, and the relative expression thereof. A shows the results before fractionation; B the results of nuclei; and C the result of the cytoplasm.
[Figure 5] Figure 5 shows the comparative results of the effect of exosome, pirfenidone (PFD) and nintedanib. Primary lung fibroblast was stimulated with individual component at the concentrations described in the figure, and then, expressions of various myofibroblast markers were confirmed by western blotting.

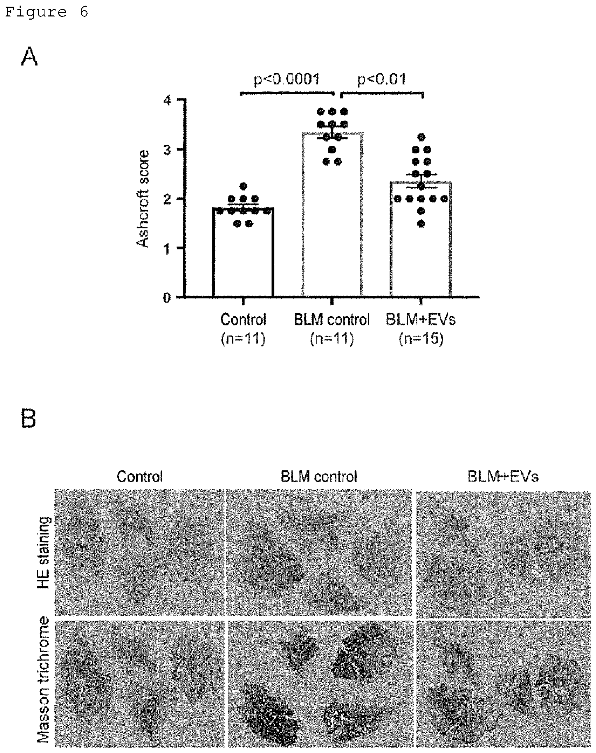

[Figure 6] Figure 6 shows (A) Ashcroft score and (B) the results of HE staining and Masson trichrome staining of untreated mice (control), pulmonary fibrosis model mice (BLM control) prepared by administering bleomycin, and pulmonary fibrosis model mice (BLM + EV) prepared by administering exosome.

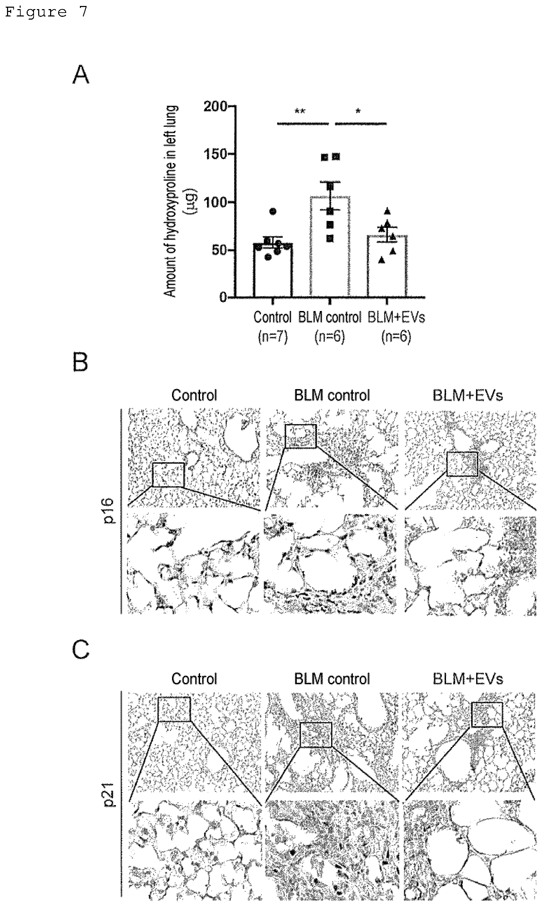

[Figure 7] Figure 7 shows (A) the amount of hydroxyproline of a left lung, and (B-C) staining results of cellular senescence markers p16 and p21 in lung tissue in untreated mice (control), pulmonary fibrosis model mice (BLM control) prepared by administering bleomycin, and pulmonary fibrosis model mice (BLM + EV) prepared by administering exosome.

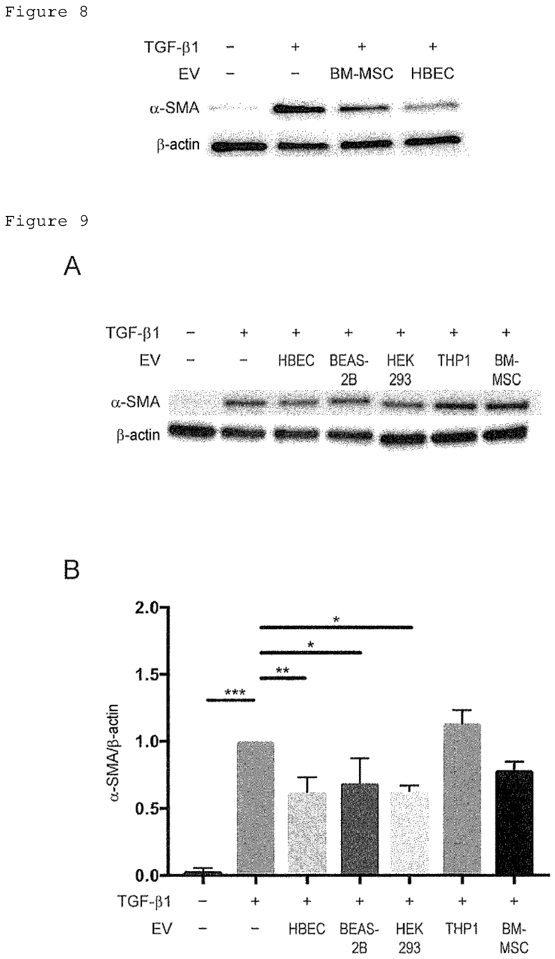

[Figure 8] Figure 8 shows the results of α-SMA expression confirmed by western blotting after primary lung fibroblast was stimulated with TGF-β1 and/or exosome (HBEC-derived exosome or bone marrow-derived mesenchymal stem cell (BM-MSC)-derived exosome).
[Figure 9] Figure 9 show (A) the results of α-SMA expression confirmed by western blotting after primary lung fibroblast was stimulated with TGF-β1 and/or various cell-derived exosome and (B) the relative expression thereof. The cell from which exosome is derived is human bronchial epithelial cell HBEC, human bronchial epithelial cell line BEAS-2B, human embryonic kidney cell 293 (HEK293), monocytic leukemia cell line THP1 and bone marrow-derived mesenchymal stem cell BM-MSC.

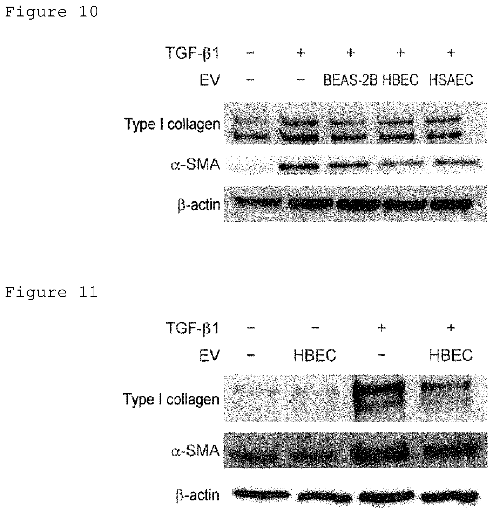

[Figure 10] Figure 10 shows expression of type I collagen and α-SMA confirmed by western blotting after primary lung fibroblast was stimulated with TGF-β1 and/or exosome (BEAS-2B-derived exosome, HBEC-derived exosome or HSAEC-derived exosome).
[Figure 11] Figure 11 shows expression of type I collagen and α-SMA confirmed by western blotting after human dermal fibroblast NHDF was stimulated with TGF-β1 and/or HBEC-derived exosome.

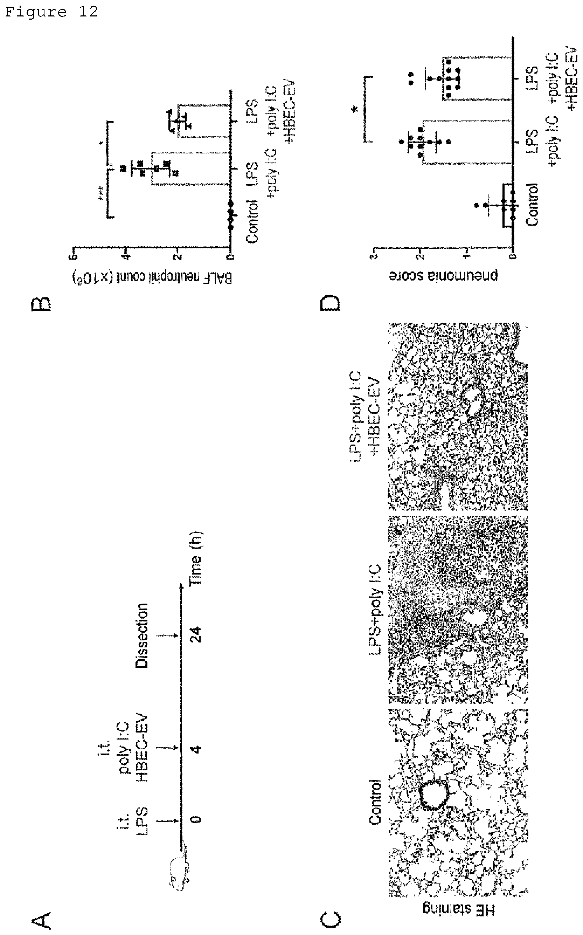

[Figure 12] Figure 12 shows (B) the results of BALF neutrophil count, (C) the results of HE staining and (D) the results of pneumonia score in untreated mice (control); acute respiratory distress syndrome (ARDS) model mice (LPS + poly I: C) prepared by spraying lipopolysaccharide (LPS) and a synthetic double strand RNA analog, poly I: C to the trachea; and ARDS model mice (LPS + poly I: C + HBEC-EV) prepared by administering HBEC-derived exosome. Reference symbol * shows a significant difference (*: p < 0.05, ***: p < 0.001).

### Description of Embodiments

### 1. Therapeutic and/or preventive agent for disease

In an embodiment, the present invention relates to a therapeutic and/or preventive agent for at least one disease of a fibrosis, an inflammatory disease and an aging disease, containing extracellular vesicle derived from cell of a tissue or a surrounding tissue thereof where the disease can occur.

In the specification, "fibrosis" refers to a disease accompanied by excessive formation of a fibrous connective tissue that can occur during a process such as repair or healing process of an organ or a tissue. Fibrosis can be a disease caused by abnormal deposition of scar tissue or a disease accompanied by abnormal deposition of scar tissue, and can occur in various tissues. Examples of fibrosis include, but are not limited to, pulmonary fibrosis, pulmonary hypertension, cirrhosis, cardiomyopathy, ischemic heart disease, valvular disease, endocardial myocardial fibrosis, cardiac fibrosis, arteriosclerosis, nephrogenic fibrosis, renal sclerosis, glomerulosclerosis, scleroderma, retroperitoneal fibrosis and uterine fibrosis.

Scleroderma is a disease having dermal sclerosis as a main symptom. There are two types, i.e., localized scleroderma and systemic scleroderma, in scleroderma. Localized scleroderma is a disease having a damage only in skin. Systemic scleroderma is a disease having a pathological change not only in skin but also in various organ of the whole body.

In the specification, "inflammatory disease" refers to a disease having persistent or transient inflammatory condition and improved by mitigating the inflammatory condition. Examples of the inflammatory disease include, but are not limited to, COPD (chronic obstructive pulmonary disease), bronchial asthma, acute exacerbation of interstitial pneumonia, ARDS (acute respiratory distress syndrome), radioactive pneumonitis, lung sarcoidosis, bronchiectasis, cystic fibrosis, bronchopulmonary dysplasia, viral hepatitis, autoimmune hepatitis, primary biliary cholangitis, primary sclerosing cholangitis, hepatocellular carcinoma, Crohn's disease, ulcerative colitis, acute pancreatitis, chronic pancreatitis, autoimmune pancreatitis, aortitis, Basedow's disease, Hashimoto's disease, diabetes, nephritis, collagenosis (for example, rheumatoid arthritis, SLE (systemic lupus erythematosus), vasculitis, Sjogren's syndrome, arthritis, sarcoidosis), dermatomyositis and psoriasis.

ARDS is a serious respiratory disorder induced by a preceding disease. Examples of the preceding disease inducing ARDS include bacterial pneumonia, interstitial pneumonia, acute exacerbation of interstitial pneumonia, sepsis, viral infection, polytrauma, burns, acute pancreatitis, poisonous-gas inhalation, drug addiction, drowning, pulmonary contusion, radiation lung damage and transfusion. Examples of the viral infection inducing ARDS include, but are not limited to, viral infections with an influenza virus and a corona virus such as COVID-19.

In the specification, "aging disease" refers to a disease occurring with a decrease of homeostasis function in tissue and individual due to aging. A typical phenotype of an aging disease is cellular senescence. The accumulation of the senescent cell and various mediator secreted from the senescent cell cause chronic inflammatory disease and are further involved in structural change such as fibrosis and dysfunction. Examples of the aging disease include, but are not limited to, Alzheimer's disease, dementia, Parkinson's disease, spinocerebellar degeneration and multiple system atrophy.

Note that, the above disease is non-mutually exclusively classified. A single disease can belong to a plurality of classifications. For example, primary sclerosing cholangitis and hepatocellular carcinoma each can be an inflammatory disease as well as a fibrosis.

The therapeutic and/or preventive agent of the present invention contains, consists of, or substantially consists of extracellular vesicle derived from cell of a tissue or a surrounding tissue thereof where a disease as mentioned above can occur.

In the specification, "extracellular vesicle (EV)" refer to vesicle released from cell. It is known that the extracellular vesicle mediates intercellular communication and are involved in various biological process such as immune response, blood coagulation and induction of phagocytosis. The extracellular vesicle is classified, based on the intracellular origin, into exosome, micro-vesicle and apoptotic body.

In an embodiment, extracellular vesicle constituting the therapeutic and/or preventive agent of the present invention is exosome or substantially exosome.

In the specification, "exosome" refers to extracellular vesicle released from various type of cell and having a diameter of about 20 to 200 nm. It is known that exosome may have various function including intercellular communication, antigen presentation and transport of protein and nucleic acid such as mRNA and miRNA.

A method for preparing extracellular vesicle or exosome is not limited; for example, a method known in the art can be used. For example, if the cell described in the specification is cultured in a medium, extracellular vesicle or exosome can be collected from the culture supernatant. The conditions (e.g., temperature and period) for the culture can be appropriately selected. For example, the culture temperature can be about 20°C to about 40°C, about 30°C to about 40°C, about 35°C to about 39°C, about 36°C to about 38°C or about 37°C. The culture period can be, for example, 6 hours to 7 days, 12 hours to 4 days, 1 day to 3 days or about 2 days. Culture may be carried out in the presence of CO₂. In this case, the concentration of CO₂ can be about 2% to about 10%, about 4% to about 6% or about 5%. The medium for use in culture can be selected depending on the type of cell. Examples of the medium that can be used include a commercially available medium (for example, BEGM, DMEM, MEM, BME, RPMI 1640, advanced RPMI 1640, F-10, F-12, DMEM-F12, α-MEM, IMDM, McCoy's 5A medium, mTeSR1 medium or a mixture of these) and a medium prepared. To a medium, various additive (for example, serum or a serum substitute, a non-essential amino acid and an antibiotic such as penicillin and streptomycin) can be added. The medium preferably contain neither extracellular vesicle nor exosome derived from other component such as serum.

A method for collecting extracellular vesicle or exosome from a culture supernatant is not limited and extracellular vesicle can be collected by a method known in the art, for example by a method of using ultracentrifugation (for example, Thery C., Curr. Protoc. Cell Biol. (2006) Chapter 3: Unit 3.22.), polymer precipitation, immunoprecipitation, FACS, ultrafiltration, gel filtration or HPLC and a method of allowing the vesicle or exosome to adhere to beads serving as carrier by use of an antibody or lectin. Extracellular vesicle or exosome may be collected by use of a commercially available isolation kit for extracellular vesicle or exosome.

Of the above collection methods, ultracentrifugation is a standard method most frequently used for isolation of extracellular vesicle or exosome. The centrifugal force employed in the ultracentrifugation is, for example 50000 × g or more, 100000 × g or more, or 150000 × g or more, and 300000 × g or less, 250000 × g or less, or 200000 × g or less. The time for centrifugation, which is not limited, can be, for example, 30 minutes to 120 minutes, 60 minutes to 90 minutes or 70 minutes to 80 minutes. Before centrifugation, if necessary, foreign substances may be removed or decreased by filtration with a filter and/or centrifugation using lower centrifugal force.

Collection of extracellular vesicle or exosome and confirmation of physical characteristic of extracellular vesicle or exosome can be carried out in accordance with a method known in the art, for example, visual confirmation by an electronic microscope or Nano Tracking Analysis (NTA) for the size and number of extracellular vesicle or exosome. Alternatively, the presence of extracellular vesicle or exosome can be confirmed by checking expression of a protein and/or gene that can serve as a marker for extracellular vesicle or exosome.

In the specification, cell from which extracellular vesicle or exosome is derived is differentiated cell. In the specification, "differentiated cell" refer to cell differentiated from undifferentiated cell such as stem cell. Examples of the "differentiated cell" in the specification include differentiated cell present in a living tissue such as blood, skin, muscle, nerve, fat, a digestive organ, a respiratory organ, a urinary organ, a genital organ and an endocrine organ. More specifically, epithelial cell, endothelial cell, mesothelial cell, muscle cell and nerve cell are mentioned.

In the specification, cell may be any of the primary cultured cell, subcultured cells and frozen cell. In the specification, cell may be affected or unaffected with disease as mentioned above. In an embodiment, cell is derived from a healthy subject. In the specification, "healthy subject" refers to an individual in a healthy state. In the specification, the healthy subject broadly includes healthy cell. Accordingly, as long as a subject is healthy not only at an individual level but also at a cellular level like a normal tissue or a normal part of a tissue collected from a patient with disease as mentioned above, the subject is called as a healthy subject.

The species of organism from which cell is derived, which is not limited, may be, for example, the same species to which the therapeutic and/or preventive agent or composition of the present invention is to be administered. Examples thereof include, but are not limited to, mammals including primates such as a human and a chimpanzee; experimental animals such as a rat and a mouse; livestock animals such as a pig, a cow, a horse, a sheep and a goat; and pet animals such as a dog and a cat. The species of organism is, for example, a human or a mouse, and preferably a human.

As mentioned above, extracellular vesicle or exosome is derived from cell of a tissue or a surrounding tissue thereof that disease as mentioned above can occur. The tissue from which cell donating extracellular vesicle is derived may be the same as or different from a tissue having cell receiving the extracellular vesicle, or they are mutually located in the surroundings of the other tissue. In an embodiment, the tissue from which the cell donating extracellular vesicle is derived is the same as a tissue having the cell receiving the extracellular vesicle. In another embodiment, the tissue from which the cell donating extracellular vesicle is derived is different from a tissue having the cell receiving the extracellular vesicle.

If a tissue from which cell donating extracellular vesicle is derived and a tissue having cell receiving the extracellular vesicle are the same or mutually located in the surroundings of the other tissue, unlimited examples of a combination of disease as mentioned above and cell of a tissue or a surrounding tissue thereof where the disease occurs include the followings:
Bronchial epithelial cell (for example, tracheal epithelial cell or bronchial epithelial cells) or alveolar epithelial cell (for example, type I alveolar epithelial cell or Type II alveolar epithelial cell)-derived extracellular vesicle can be used for disease that may occur in lung or airway (for example, pulmonary fibrosis, pulmonary hypertension, COPD, bronchial asthma, ARDS, radioactive pneumonitis, lung sarcoidosis, alveolar bleeding derived from collagenosis (for example, SLE (systemic lupus erythematosus) or vasculitis); interstitial pneumonia accompanied with collagenosis (for example, SLE, vasculitis, scleroderma, Sjogren's syndrome, rheumatoid arthritis, sarcoidosis); bronchiectasis; cystic fibrosis; and bronchopulmonary dysplasia);
extracellular vesicle derived from epithelial cell of eye can be used for disease that may occur in eye (for example, diabetic retinopathy);
extracellular vesicle derived from the epithelial cell of ear, nose and throat can be used for disease that may occur in ear, nose and throat (for example, chronic sinusitis, chronic otitis media, cholesteatoma, nasal polyps);
extracellular vesicle derived from kidney epithelial cell can be used for disease that may occur in kidney or glomerulus (for example, nephritis, nephrogenic fibrosis, renal sclerosis, glomerulosclerosis, diabetic nephropathy and nephritis accompanied by SLE and scleroderma;
extracellular vesicle derived from liver epithelial cell, bile duct epithelial cell or gallbladder epithelial cell can be used for disease that may occur in liver, gall bladder or bile duct (for example, viral hepatitis, autoimmune hepatitis, primary biliary cholangitis, primary sclerosing cholangitis, cirrhosis, hepatocellular carcinoma);
extracellular vesicle derived from intestinal epithelial cell (for example, small intestine or large intestine epithelial cell) can be used for disease that may occur in intestinal tract (for example, Crohn's disease and ulcerative colitis);
extracellular vesicle derived from pancreatic epithelial cell can be used for disease that may occur in pancreas (for example, acute pancreatitis, chronic pancreatitis and autoimmune pancreatitis);
extracellular vesicle derived from cardiac epithelial cell or cardiomyocytes can be used for disease that may occur in heart (for example, cardiomyopathy, ischemic heart disease, valvular disease, endocardial myocardial fibrosis and cardiac fibrosis);
extracellular vesicle derived from vascular epithelium or endothelial cell can be used for disease that may occur in blood vessel (for example, arteriosclerosis, aortitis and vascular lesions accompanied by diabetes);
extracellular vesicle derived from thyroid epithelial cell can be used for disease that may occur in thyroid (for example, Basedow's disease and Hashimoto's disease);
extracellular vesicle derived from brain epithelial cell, ependymal cell or nerve cell can be used for disease that may occur in brain or nerve (for example, Alzheimer's disease, dementia, Parkinson's disease, spinocerebellar degeneration, multiple system atrophy and diabetic neuropathy);
extracellular vesicle derived from peritoneal epithelial cell can be used for disease that may occur in abdominal cavity (for example, retroperitoneal fibrosis);
extracellular vesicle derived from uterine epithelial cell can be used for disease that may occur in uterus and ovary (for example, uterine fibrosis, ovarian tumor);
extracellular vesicle derived from epithelial cell of skin can be used for disease that may occur in skin (for example, scleroderma, dermatomyositis); and
extracellular vesicle derived from bone cell or chondrocytes can be used for disease that may occur in bone or joint (for example, rheumatoid arthritis).

Note that, scleroderma may occur in various organs and dermatomyositis may be caused in muscle. In these cases, extracellular vesicle derived from the cells of respective tissues can be used.

If the tissue from which cell donating extracellular vesicle is derived is different from a tissue having cell receiving the extracellular vesicle, a combination of a disease as mentioned above and a tissue or a surrounding tissue thereof where the disease can occur is not limited.

For example, extracellular vesicle derived from cell present in organ except lung, for example, kidney cell-derived extracellular vesicle (for example, extracellular vesicle derived from renal epithelial cell) can be used for disease that may occur in lung or airway;
extracellular vesicle derived from cell present in organ except eye can be used for disease that may occur in eye;
extracellular vesicle derived from cell present in organ except ear, nose and throat can be used for disease that may occur in ear, nose and throat;
extracellular vesicle derived from cell present in organ except the kidney or glomerulus, for example, extracellular vesicle derived from cell in lung (for example, extracellular vesicle derived from bronchial epithelial cell, tracheal epithelial cell, bronchial epithelial cell or alveolar epithelial cell) can be used for disease that may occur in kidney or glomerulus;
extracellular vesicle derived from cell present in organ except liver can be used for disease that may occur in liver, gall bladder or bile duct;
extracellular vesicle derived from cell present in organ except the intestinal tract can be used for disease that may occur in intestinal tract;
extracellular vesicle derived from cell present in organ except pancreas can be used for disease that may occur in pancreas;
extracellular vesicle derived from cell present in organ except heart can be used for disease that may occur in heart;
extracellular vesicle derived from cell present in organ except blood vessel can be used for disease that may occur in blood vessel;
extracellular vesicle derived from cell present in organ except thyroid can be used for disease that may occur in thyroid;
extracellular vesicle derived from cell present in organ except brain or nerve can be used for disease that may occur in brain or nerve;
extracellular vesicle derived from cell present in organ except abdominal cavity can be used for disease that may occur in abdominal cavity;
extracellular vesicle derived from cell present in organ except uterus can be used for disease that may occur in uterus;
extracellular vesicle derived from cell present in organ except ovary can be used for disease that may occur in ovary;
extracellular vesicle derived from cell present in organ except skin, for example, kidney cell-derived extracellular vesicle (for example, extracellular vesicle derived from renal epithelial cell) and lung cell-derived extracellular vesicle (for example, extracellular vesicle derived from the bronchial epithelial cells, tracheal epithelial cell, bronchial epithelial cell or alveolar epithelial cell) can be used for disease that may occur in skin; and
extracellular vesicle derived from cell present in organ except bone or joint, can be used for disease that may occur in bone or joint.

Note that, scleroderma can occur in various organs and dermatomyositis can occur in muscle. In these cases, extracellular vesicle derived from cell of tissue except the tissue affected with scleroderma can be used.

In the specification, "treatment" includes complete or partial cure, mitigation or prevention of progression of disease in a patient already having symptom or symptom accompanied by the disease. In the specification, "prevention" include suppression of manifestation of a disease in a patient that may suffer from disease, or reduction of an incidence rate of disease.

### 2. Composition

In an embodiment, the present invention relates to a composition containing extracellular vesicle or exosome, or a therapeutic and/or preventive agent described in the specification, for treating and/or preventing at least one disease of a fibrosis, an inflammatory disease and an aging disease, for example, a pharmaceutical composition, a food composition or a supplement. The composition of the present invention may substantially consist of extracellular vesicle or exosome, or a therapeutic and/or preventive agent described in the specification.

The amount of extracellular vesicle or exosome (for example, effective amount for treatment and/or prevention) contained in composition of the present invention can be appropriately determined by those skilled in the art in consideration of various factors including the gender, body weight and age of the subject, and disease process and symptom. For example, the amount of extracellular vesicle or exosome contained in composition of the present invention may be, for example, about 0.0001 to 100.0 mg, about 0.001 to 10.0 mg, about 0.01 to 1.0 mg or about 0.05 to 2.0 mg per body weight (1 kg) of the subject to which composition is to be administered, but are not limited to this.

Composition of the present invention may contain, in addition to the above extracellular vesicle or exosome or a therapeutic and/or preventive agent, other component and a carrier such as a pharmaceutically acceptable carrier, for example sterilized water, saline, a buffer, an excipient, a binder, a disintegrant, an emulsifier, a surfactant, a stabilizer, a lubricant, a diluent, a streaming accelerator, a flavoring agent, a colorant and a fragrance.

Composition of the present invention can be formulated in accordance with a routine method. The formulation may be performed with reference to a method described in, for example, Remington's Pharmaceutical Sciences (Merck Publishing Co., Easton, Pa.)

The dosage form is not particularly limited and appropriately selected where necessary. The composition can be administered generally as an oral preparation such as a tablet, a capsule, a granule, a fine granule, a powder, a liquid, a syrup, a suspension, an emulsion and an elixir, or as a parenteral agent such as an injection, an intravenous agent, a suppository, an inhalant, a transdermal absorbent, a transmucosal absorbent, a patch and an ointment.

The administration route of composition of the present invention is not limited but composition may be administered through inhalation, aerosol, injection, intravenous injection, oral, percutaneous, transnasal, local, transvaginal, transmucosal or transrectal route. Composition of the present invention may be formulated for inhalation, aerosol, injection, intravenous injection, oral, percutaneous, transnasal, local, transvaginal, transmucosal or transrectal administration. The route of administration can be appropriately selected depending on the disease; for example, in the case of a lung disease, inhalation or aerosol administration can be employed.

Examples of the subject to which the therapeutic and/or preventive agent or composition of the present invention is to be administered, include, but are not limited to, mammals including primates such as a human and a chimpanzee; experimental animals such as a rat and a mouse; livestock animals such as a pig, a cow, a horse, a sheep and a goat; and pet animals such as a dog and a cat. The subject is, for example, a human or a mouse, and preferably a human.

The dose, administration interval and administration period of extracellular vesicle or exosome, a therapeutic and/or preventive agent or a composition described in the specification, can be appropriately determined by those skilled in the art in consideration of various factors including the gender, body weight and age of the subject, and disease process and symptom. For example, the dosage of extracellular vesicle or exosome, a therapeutic and/or preventive agent or a composition may be, for example, about 0.0001 to 100.0 mg/1 kg body weight, about 0.001 to 10.0 mg/1 kg body weight, about 0.01 to 1.0 mg/1 kg body weight or about 0.05 to 2.0 mg/1 kg body weight. The frequency of administration is three times a day, twice a day, once a day, once every two days, once every three days, once a week, once every two weeks or once every month, but is not limited to this. The administration period, which is not limited, may be one day, two days, three days, one week, two weeks, one month, half a year, or one year or more.

### 3. Other embodiments

In an embodiment, the present invention relates to a method for treating and/or preventing at least one disease of a fibrosis, an inflammatory disease and an aging disease, including administering extracellular vesicle or exosome, a therapeutic and/or preventive agent or composition described in the specification to a subject. The administration target, dosage, frequency of administration and route of administration are the same as described in the above section "2. Composition".

In an embodiment, the present invention relates to extracellular vesicle or exosome, therapeutic and/or preventive agent or composition described in the specification for use in treating and/or preventing at least one disease of a fibrosis, an inflammatory disease and an aging disease.

The present invention will be more specifically described with reference to Examples hereafter, but the scope of the present invention is not limited by Examples.

### Examples

### <Materials and methods>

The test protocol used herein was approved by the institutional ethics committee of the Jikei University School of Medicine. Written consents with respect to all materials used herein were obtained.

### (Sample: human primary bronchial epithelial cell and human primary lung fibroblast)

Human primary bronchial epithelial cell (HBEC) and human primary lung fibroblast were both obtained by isolation culture of a tissue excised out from lung in the hospital of the Jikei University School of Medicine. The isolation culture was carried out in accordance with the method described in the report (Araya J. et al., 2007, J. Clin. Invest., 117, pp. 3551 to 3562). Human bronchial epithelial cell line BEAS-2B was purchased from ATCC (Manassas, USA); human small airway epithelial cell HSAEC from Lonza; bone marrow-derived mesenchymal stem cell BM-MSC form RIKEN; human embryonic kidney cell 293 (HEK293) from ATCC; monocytic leukemia cell line THP1 form ATCC and human dermal fibroblast NHDF from Lonza.

### (Cell culture)

Cell was maintained in a medium containing 10% heat-inactivated fetal bovine serum (FBS) and Antibiotic-Antimycotic (Thermo Fisher Scientific) at 37°C and 5%CO₂. BEAS-2B was cultured in Advanced RPMI 1640 medium (Thermo Fisher Scientific); human primary bronchial epithelial cell in BEGM medium (Lonza); human primary lung fibroblast in DMEM medium (Thermo Fisher Scientific); HSAEC in SAGM medium (Lonza); BM-MSC in MessenPRO medium (Thermo Fisher Scientific); HEK293 in RPMI 1640 medium (Thermo Fisher Scientific); THP1 in RPMI 1640 medium

### (Thermo Fisher Scientific); and NHDF in DMEM medium (Thermo Fisher Scientific).

### (Exosome collection method)

Human bronchial epithelial cell line BEAS-2B was cultured for two days in Advanced RPMI 1640 medium (Thermo Fisher Scientific); human primary bronchial epithelial cell HBEC in BEGM medium (Lonza); HSAEC in SAGM medium (Lonza); BM-MSC in STEMPRO medium (Thermo Fisher Scientific); HEK293 in Advanced DMEM medium (Thermo Fisher Scientific) and THP1 in Advanced RPMI 1640 medium (Thermo Fisher Scientific), and culture supernatants were collected. Each of the culture supernatants collected was filtered by a 0.22 µm filter (Millipore), and then, subjected to ultracentrifugation (35,000 rpm, 70 minutes, 4°C, SW41Ti rotor, company: Beckman) to separate/purify exosome. Note that, ultracentrifugation is a technique for ultracentrifuging sample and used for precipitating/isolating exosome, and most frequently and normally used for isolating exosome. The size and number of the exosome obtained were measured by use of a technology called NTA (Nano Tracking Analysis) (NanoSight).

### (Western blotting)

Protein collection from human primary bronchial epithelial cell, human primary lung fibroblast and human dermal fibroblast, which were added exosome and/or transforming growth factor-β (TGF-β) 1, was carried out by use of Mammalian Protein Extract Reagent (M-PER, Thermo Fisher Scientific). To the sample thus obtained, a sample buffer (Wako, 198-13282 or 191-13272) was added, and then, the sample was subjected to electrophoresis, i.e., SDS-PAGE. Subsequently, transferring bands to a membrane (polyvinylidene difluoride membrane, Millipore) and blocking with Blocking One (Nacalai Tesque) were carried out. After an antigen-antibody reaction was carried out, light was allowed to emit from blots by use of ImmunoStar LD (Wako), and quantitative determination was carried out. As a primary antibody, alpha smooth muscle actin (α-SMA, Sigma-Aldrich, A2547), goat anti-COL1/type I collagen (Southern Biotech, 1310-01), mouse anti-FN1/cellular fibronectin containing extra domain A (Abcam, ab6328), mouse anti-ACTB/b-actin (Sigma-Aldrich, A5316), rabbit anti-p21^{Waf1/Cip1} (Cell Signaling Technology, 2947), rabbit anti-β-Catenin (Cell Signaling Technology, 8480) and rabbit anti-non-phospho (Active) β-Catenin (Cell Signaling Technology, 19807) were used. The intensity values of the bands obtained were determined by Image J.

### (Staining of senescence associated β-galactosidase)

To human primary bronchial epithelial cell, TGF-β1 and/or bronchial epithelial cell-derived exosome were added and the number of positive cell to staining was counted by Senescence associated β-galactosidase staining kit (Sigma, CS0030). Staining was carried out in accordance with the instructions provided by the manufacturer.

### (Cell-component fractionation by WNT/b-catenin signaling analysis)

Nucleus and cytoplasmic proteins were fractionated by a cell component fractionation kit (Nuclear and Cytoplasmic Extraction Reagents, Thermo Fisher Scientific, 78833) in accordance with the instructions provided by the manufacturer.

### (Animal experiment)

Animal experiments were conducted in accordance with the guidelines of the Laboratory Animal Research Institute of the Jikei University School of Medicine. Pulmonary fibrosis model mice were prepared by administering 2.5 U/kg bleomycin (Nippon Kayaku Co., 4234400D4032) dissolved in 50 µl of saline through the airway to 8-12 weeks-old C57BL/6J mice, and put in use. Day 7 and 14 after administration of bleomycin, BEAS2B-derived exosome (3 µg (50 µl)) were administered through the airway. Day 17 after administration, the mice were sacrificed and tissues thereof were used for immunostaining.

### (Immunostaining of tissue)

A mouse lung was stained with Masson trichrome, hematoxylin and eosin and evaluated as described in the report (Kobayashi K. et al., J. Immunol., 2016, 197 (2), pp. 504-16). The tissues were stained with Masson trichrome and Ashcroft scores were determined in accordance with the Ashcroft's method to evaluate fibrosis. For histological staining of cellular senescence marker p16, an anti-mouse CDKN2A/p16INK4a antibody (abcam, ab54210) was used as a primary antibody. For histological staining of cellular senescence marker p21, rabbit anti-p21^{Waf1/Cip1} (Cell Signaling Technology, 2947) was used as a primary antibody.

The amount of hydroxyproline was measured by the hydroxyproline assay kit (Chondrex, 6017).

### (Statistical analysis)

An experiment was repeated at least three times and an average ± SEM was used as the result. Student t test was used for comparison of three data items and ANOVA was used for comparison of more than three data items. Prism version 7 (GraphPad Software, San Diego, CA) was used and p value < 0.05 was regarded as a statistically significant difference.

### (Comparison between HBEC-derived exosome and bone marrow-derived MSC-derived exosome)

TGF-β1 (2 ng/ml) and/or exosome (10 µg/ml) (HBEC-derived exosome or bone marrow-derived mesenchymal stem cell (BM-MSC)-derived exosome) were added to primary lung fibroblast. The mixture was cultured for 24 hours.

After the culture medium was exchanged, the mixture was cultured for further 48 hours. Thereafter, western blotting was carried out in the same manner as in the above. BM-MSC-derived exosome was collected by culturing BM-MSC in StemPro MSC SFM (Thermo Fisher Scientific) and subjecting the culture supernatant to ultracentrifugation in the same manner as in the case of HBEC-derived exosome described above.

### (ARDS model mouse)

Lipopolysaccharide (LPS), which is a component of a gram-negative bacterium and used in a sepsis model, and a synthetic double-strand RNA analog, Poly I: C, which mimics virus RNA, were directly sprayed to the trachea of each of mice (C57BL/6J, 8 weeks old, male). In this manner, acute respiratory distress syndrome (ARDS) model mice were prepared. To describe more specifically, LPS (5 µg/mg) was sprayed. Four hours later, Poly I: C (5 µg/mg) was sprayed. Further 20 hours later, the mice were sacrificed, and then, the number of BALF neutrophils was determined, HE staining was carried out and pneumonia score was determined. When HBEC-derived exosome was used, Poly I: C was sprayed; at the same time, 2 × 10⁹ exosome particles per mouse were sprayed through the airway (Figure 12A).

The count of neutrophils in BALF (bronchoalveolar lavage fluid) was obtained by the following method. First, the total count of cell in BALF was obtained by a method using trypan blue. Then, BALF was centrifuged by an auto smear centrifuge (CF-120, 800 rpm, 5 minutes) and the resultant supernatant was subjected to staining with Diff-Quik. The percentage of the neutrophil count relative to the total cell count was obtained. Based on the percentage, the count of neutrophils in BALF was calculated.

Pneumonia score was determined by the following method with reference to the method described in Sun Y. Q. et al., Stem Cells, 30 (12); 2692-9 (2012). The lung tissue of a single mouse was sectioned. In the tissue slice around the bronchi, 5 regions were selected at random. In each of the regions, the infiltration with inflammatory cell around bronchi and vascular was scored on 4 points scale from 0 to 3. The scores of the 5 regions were averaged and used as the pneumonia score.

### <Results>

### (Analysis of exosome prepared)

The culture supernatants of human primary bronchial epithelial cell HBEC and human bronchial epithelial cell line BEAS-2B were subjected to ultracentrifugation. The exosome thus collected were analyzed by NTA. The analysis results (A: HBEC, B: BEAS-2B) are shown in Figure 1.

These results show that exosome was ultracentrifugally collected without trouble. The average diameter of the collected exosome was about 80 nm.

### (Suppression of differentiation of TGF-β1-induced myofibroblast by exosome)

In pathogenesis of airway remodeling of a disease such as pulmonary fibrosis, COPD and bronchial asthma and the like, differentiation into myofibroblast plays an important role. Then, the effect of exosome on lung fibroblast was investigated.

TGF-β1 (2 ng/ml) and/or 10 µg/ml exosome (HBEC-EV) were added to primary lung fibroblast. Twenty-four hours later, the culture medium was exchanged. The fibroblast was cultured for further 48 hours. Thereafter, expression of myofibroblast markers, i.e., α-SMA, type I collagen and fibronectin, and a housekeeping protein, i.e., actin, were confirmed by western blotting. The results are shown in Figure 2A. The expression levels of individual marker proteins were divided by the expression level of actin. The resultant values are shown in Figures 2B to D. The TGF-β1-induced increase in each of α-SMA, type I collagen and fibronectin expression was decreased by addition of exosome. From this, it was demonstrated that HBEC-derived exosome suppress TGF-β1-induced myofibroblast differentiation.

### (Suppression of senescence of TGF-β1 induced lung epithelial cell by exosome)

Recently, it has been widely known that senescence of pulmonary epithelial cell is involved in pathological condition of pulmonary fibrosis and COPD. The inventors investigated the effect of exosome on senescence of lung epithelial cell.

Bronchial epithelial cell (HBEC)-derived exosome (10 µg/ml) was added together with TGF-β1 (2 ng/ml) to bronchial epithelial cell (HBEC) and the mixture was incubated for 48 hours. As a result, TGF-β1-induced increase in expression of aging markers, SA-β-Gal (senescence associated β-galactosidase) (Figure 3A) and p21 (Figure 3B), was suppressed. When human small airway epithelial cell (HSAEC)-derived exosome was used, TGF-β1-induced increase in expression of p21 was suppressed similarly to the case of HBEC-derived exosome (Figure 3C). The suppressive effect of HBEC-derived exosome on the increase of p21 expression was larger than that of BM-MSC-derived exosome (Figure 3D).

Next, the mechanism of action to suppress cellular senescence by exosome was investigated. So far, it is known that myofibroblast differentiation in pulmonary fibrosis is suppressed by suppressing WNT/b-catenin signal.

Then, the influence of bronchial epithelial cell-derived exosome on WNT/b-catenin was investigated. As a result, exosome suppressed TGF-β1-induced WNT/b-catenin. The result suggests that exosome suppress TGF-β1-induced myofibroblast differentiation through suppression of WNT/b-catenin (Figure 4, A: before fractionation, B: nucleus, C: cytoplasm).

From the above results, it was demonstrated that bronchial epithelial cell-derived exosome can exert a therapeutic and/or preventive effect on disease such as pulmonary fibrosis by suppressing the differentiation of myofibroblast and/or the senescence of epithelial cell.

### (Comparison with existing therapeutic agents)

Comparison with the effects of existing therapeutic agents for idiopathic pulmonary fibrosis, pirfenidone and nintedanib, was made.

As comparative subjects, pirfenidone was used at a blood concentration of 10 µg/ml and a concentration of 500 µg/ml (50 fold as large as the blood concentration), and nintedanib was used at a blood concentration of 100 nM, and a concentration of 5000 µg/ml (50 fold as large as the blood concentration). Primary lung fibroblast was stimulated with each component at the concentrations described above for 24 hours. After the culture medium was exchanged, the fibroblast was cultured for further 48 hours and subjected to analysis.

The results are shown in Figure 5. Bronchial epithelial cell line-derived exosome suppressed TGF-β1-induced myofibroblast differentiation markers, type I collagen, fibronectin (FN) and α-SMA, in a concentration dependent manner, and the suppressive effect of the exosome was equal to or larger than that produced by an IPF therapeutic agent (blood concentration).

### (Animal experiment)

To 8-12 weeks-old C57BL/6J mice, 2.5 U/kg bleomycin was administered through the airway. In this manner, pulmonary fibrosis model mice were prepared. When bronchial epithelial cell line-derived exosome was administered through the airway to the model mice, lung fibrosis score, which was confirmed by staining with Masson trichrome, decreased (Figures 6A and B). Further, when bronchial epithelial cell line-derived exosome was administered through the airway, the amount of hydroxyproline in lung tissue, which serves as an indicator of collagen accumulation, decreased (Figure 7A), and the counts of cell senescence markers p16- and p21-positive cell decreased (Figures 7B and C). Accordingly, it was demonstrated that the bronchial epithelial cell line-derived exosome suppresses fibrosis and aging in pulmonary fibrosis mouse *in vivo.*

### (Comparison with MSC-derived exosome)

TGF-β1 and/or exosome (HBEC-derived exosome or bone marrow-derived mesenchymal stem cell (BM-MSC)-derived exosome) were added to primary lung fibroblast. The mixture was cultured for 24 hours. After the culture medium was exchanged, the mixture was cultured for further 48 hours. Thereafter, expression of α-SMA serving as a marker for myofibroblast and actin serving as a housekeeping protein were confirmed by western blotting. The results are shown in Figure 8. As is apparent from Figure 8, the TGF-β1-induced increase in α-SMA expression was significantly reduced by addition of HBEC-derived exosome, compared to BM-MSC-derived exosome. The result indicates that more differentiated HBEC-derived exosome may have a higher ability to suppress myofibroblast differentiation in lung than undifferentiated BM-MSCs.

### (Effect of exosome derived from cell except bronchial epithelial cell)

The effect of exosome derived from cell except bronchial epithelial cell (HBEC) on TGF-β1-induced myofibroblast differentiation was investigated by adding the exosome to primary lung fibroblast. More specifically, the effects of exosome, which was derived from human bronchial epithelial cell (HBEC), a human bronchial epithelial cell line (BEAS-2B), human embryonic kidney cell line 293 (HEK293), a monocytic leukemia cell line (THP1) and human small airway epithelial cell (HSAEC), and exosome, which was derived from bone marrow-derived mesenchymal stem cells (BM-MSC), on TGF-β1-induced myofibroblast differentiation were investigated.

BEAS-2B-derived exosome and HEK293-derived exosome showed the same suppression effect as HBEC-derived exosome on the TGF-β1-induced increase in α-SMA expression. The effects of these exosomes were all more remarkable than those of BM-MSC-derived exosome (Figures 9A and B). In contrast, the effect of THP1-derived exosome was not found. BEAS-2B-derived exosome and HSAEC-derived exosome showed the same suppression effects as HBEC-derived exosome on the TGF-β1-induced increase in α-SMA expression and type I collagen expression (Figure 10) .

These results demonstrated that exosome derived from cell except bronchial epithelial cell, such as renal cell and alveolar epithelial cell, may also have a high ability to suppress myofibroblast differentiation in lung.

### (Suppression effect of exosome on TGF-β1-induced myofibroblast differentiation of dermal fibroblast)

Whether HBEC-derived exosome also has the effect of suppressing fibrosis of dermal fibroblast was investigated.

TGF-β1 (2 ng/ml) and/or exosome (HBEC-EV) (10 µg/ml) were added to dermal fibroblasts (NHDF). Twenty-four hours later, the culture medium was exchanged. The mixture was cultured for further 48 hours. Thereafter, expression of myofibroblast markers, α-SMA and type I collagen, and actin were confirmed by western blotting. The results are shown in Figure 11.

The TGF-β1-induced increase in α-SMA expression and type I collagen expression in dermal fibroblasts (NHDF) was decreased by addition of exosome. From this, it was shown that HBEC-derived exosome suppresses TGF-β1-induced myofibroblast differentiation in dermal fibroblast. This result demonstrates that lung-derived exosome may be a therapeutic agent for scleroderma in tissue different from lung.

### (Effect of exosome on the ARDS model)

Whether HBEC-derived exosome has effect of suppressing inflammatory response in acute respiratory distress syndrome (ARDS) model mice was investigated.

Lipopolysaccharide (LPS) was sprayed in the trachea. Four hours later, poly I: C and/or exosome (HBEC-EV) were sprayed in the trachea. In this manner, acute respiratory distress syndrome (ARDS) model mice were prepared. Further 20 hours later, the mice were dissected (Figure 12A) and inflammatory reaction in lung was investigated.

The increase in neutrophil count induced by LPS and poly I: C was significantly suppressed by HBEC-derived exosome (Figure 12B). Pathological images evaluated by HE staining showed that infiltration of inflammatory cell around the airway and blood vessel are reduced by HBEC-derived exosome and lung damage is suppressed (Figure 12C). In addition, increased pneumonia score induced by LPS and poly I: C was significantly decreased by HBEC-derived exosome (Figure 12D). The above results demonstrate that exosome of the present invention may be used as therapeutic agents for inflammatory disease including ARDS.

All publications, patents and patent applications cited in the specification are incorporated herein in their entirety by reference.

## Claims

1. A therapeutic and/or preventive agent for at least one disease of a fibrosis, an inflammatory disease and an aging disease, comprising extracellular vesicles derived from a cell of a tissue where the disease can occur or a surrounding tissue thereof, wherein the cell is a differentiated cell.

2. The therapeutic and/or preventive agent according to claim 1, wherein the tissue from which a cell donating the extracellular vesicle are derived is different from a tissue having a cell receiving the vesicle.

3. The therapeutic and/or preventive agent according to claim 1, wherein the cell is epithelial cell, endothelial cell, mesothelial cell, muscle cell or nerve cell.

4. The therapeutic and/or preventive agent according to claim 3, wherein the epithelial cell is bronchial epithelial cell, small airway epithelial cell or alveolar epithelial cell.

5. The therapeutic and/or preventive agent according to claim 1, wherein the tissue or surrounding tissue is selected from the group consisting of lung or airway; eye; kidney or glomerulus; liver, gallbladder or bile duct; intestinal tract; pancreas; heart; blood vessel; thyroid; brain or nerve; intraperitoneal; uterus; skin; muscle; bone; and joint.

6. The therapeutic and/or preventive agent according to claim 5, wherein the tissue or surrounding tissue thereof is lung or airway.

7. The therapeutic and/or preventive agent according to claim 4 or 6, wherein the disease is selected from the group consisting of scleroderma, pulmonary fibrosis, COPD (chronic obstructive pulmonary disease), bronchial asthma, ARDS (acute respiratory distress syndrome), pulmonary hypertension, radioactive pneumonitis, lung sarcoidosis, alveolar bleeding accompanied by collagenosis, interstitial pneumonia, bronchiectasis, cystic fibrosis or bronchopulmonary dysplasia.

8. The therapeutic and/or preventive agent according to any one of claims 1 to 7, wherein the cell is derived from a healthy subject.

9. The therapeutic and/or preventive agent according to any one of claims 1 to 8, wherein the extracellular vesicle is exosome.

10. A pharmaceutical composition comprising the therapeutic and/or preventive agent according to any one of claims 1 to 9, as an active ingredient.

11. The pharmaceutical composition according to claim 10, wherein the composition is formulated for inhalation, aerosol, injection, intravenous injection, oral, transdermal, transnasal, local, transvaginal, transmucosal or transrectal administration.
